# EUROPÄISCHE PATENTSCHRIFT

(11) **EP 1 150 662 B1**
(45) Veröffentlichungstag und Bekanntmachung des Hinweises auf die Patenterteilung: **17.09.2003**
(21) Anmeldenummer: 00907516.9
(22) Anmeldetag: 08.02.2000
(51) Int. Cl.: A61K 9/70

(54) **WIRKSTOFFHALTIGE LAMINATE FUR TRANSDERMALSYSTEME**
LAMINATES CONTAINING AN ACTIVE SUBSTANCE FOR TRANSDERMAL SYSTEMS
STRATIFIES CONTENANT UN PRINCIPE ACTIF POUR SYSTEMES TRANSDERMIQUES

(30) Priorität: 10.02.1999 DE 19906152
(43) Veröffentlichungstag der Anmeldung: 07.11.2001
(73) Patentinhaber: Jenapharm GmbH & Co. KG, 07745 Jena (DE)
(72) Erfinder: GRAWE, Detlef, D-91510 Kleinromstedt (DE); HÖSEL, Peter, D-07745 Jena (DE); FISCHER, Wilfried, D-85579 Neubiberg (DE)
(74) Vertreter: Cramer, Eva-Maria
(86) Internationale Anmeldenummer: EP0000983
(87) Internationale Veröffentlichungsnummer: WO00047191

(56) Entgegenhaltungen:
- US-A- 5 230 898

## Beschreibung

Die Erfindung betrifft wirkstoffhaltige Laminate für Transdermalsysteme.

Die Behandlung chronischer Erkrankungen mittels transdermaler Verabreichung hochwirksamer Arzneistoffe ist eine anerkannte und von Patienten gut akzeptierte Behandlungsmethode. Speziell die Behandlung von Hormondefiziten mit Estrogenen oder Testosteron stellt neben der Applikation von starken Analgetika ein rasch wachsendes Segment des pharmazeutischen Marktes dar.
Die gemeinsame Problematik aller transdermaler Applikationsformen ist es, die Diffusionsbarriere der Haut zu überwinden und therapeutisch effektive Blutspiegel aufrecht zu erhalten.
Neben Cremes und Salben werden sehr häufig transdermale Systeme, wie wirkstoffhaltige Pflaster, Bandagen o. ä., eingesetzt. Fachleuten des Gebietes der transdermalen Wirkstoffapplikation sind solche Systeme, die in unterschiedlichen technischen Ausführungsformen, wie Matrix- oder Reservoirsysteme, angeboten werden, bekannt. Allgemein enthalten Reservoirsysteme Lösungen von pharmazeutischen Wirkstoffen in flüssigen, leicht flüchtigen Lösemitteln, wie Ethanol, welche die Permeation durch intakte Haut erhöhen. Im Gegensatz dazu verzichten die "Drug-in-Adhesive-" oder Matrixsysteme auf die Verwendung dieser flüchtigen Lösemittel und verwenden Resorptionsförderer, die sich in der Matrix lösen und während des Herstellungsprozesses nicht verflüchtigen.

Absorptionsbeschleuniger für Matrixsysteme sind typischerweise lipophile schwer flüchtige Flüssigkeiten, wie Fettsäureester, mittel- oder langkettige Alkohole, Emulgatoren, Terpene o. ä. Ihre Wirkungsweisen beruhen auf folgenden wesentlichen Mechanismen:
a) Sie stören die diffusionsbehindernden Strukturen der Lipiddoppelschichten des Stratum corneum oder
b) sie erhöhen die Konzentrationen/Sättigungskonzentrationen der Arzneistoffe im Stratum corneum.
Als Resultat erhöhen beide Mechanismen den Wirkstoffflux durch die Haut. Insbesondere, wenn der Aufbau der Hautstrukturen gestört oder zerstört wird, um Permeationen zu verbessern, reagiert die Haut mit unerwünschten Nebenwirkungen, wie Hautrötungen, Jucken, Entzündungen und dergleichen. In schweren Fällen treten sogar allergische Reaktionen auf. Nur sehr wenige im Handel befindliche Transdermalsysteme enthalten keine Resorptionsbeschleuniger. Grundlage der Diffusionen durch Membranen - wie Haut - sind die Fick'schen Diffusionsgesetze. Entsprechend dieser Gesetzmäßigkeit wird die Permeation durch Membranen durch den Konzentrationsgradienten des diffundierenden Stoffes entlang des Querschnittes der Membrane bestimmt. Der Gradient wird durch die Konzentrationsdifferenz des Wirkstoffes im Donorkopartiment (transdermales System) und Haut gebildet. Hierbei wird vereinfachend die Haut als ideale homogene Membrane betrachtet. Demzufolge kann die Hautpermeation u.a. durch Variation der Wirkstoffkonzentrationen in Transdermalsystemen verändert werden. Die in der überwiegenden Zahl der Anwendungen notwendige Erhöhung der Hautpermeation hat ihre Limitierungen in
a) der Sättigungslöslichkeit des betreffenden Arzneistoffes in der zur Herstellung des Transdermalsystemes verwendeten Klebstoff-Wirkstoff-Lösung oder
b) der Sättigungslöslichkeit in der haftklebenden Matrix.

Im folgenden werden beide Fälle separat beschrieben:
A) In der überwiegenden Anzahl der Fälle werden Arzneistoffe in den Lösungen der Klebstoffe in organischen Lösemitteln, wie Ethylacetat, Aceton, Hexan oder Heptan entweder direkt oder unter Zusatz kompatibler Lösemittel gelöst. Die Lösemittelmenge, die den Klebstofflösungen zugesetzt werden kann, um Wirkstoffe darin vorzulösen, ist durch die Verträglichkeit mit den infrage kommenden Klebstofflösungen oder durch die entstehende Verdünnung des Kleberfeststoffgehaltes limitiert. In der Praxis können mittels der dem Fachmann bekannten Beschichtungsverfahren nur Klebstofflösungen mit definierten minimalen Viskositäten und Feststoffgehalten verarbeitet werden.
   Insbesondere apolare Lösemittel, die für apolare Polymere, wie Polyisobutylen, zur Verarbeitung eingesetzt werden, besitzen nur geringes Lösungsvermögen für Wirkstoffe wie Sexualhormone, Antihypertensiva, Analgetika, etc. In der Regel reicht das Lösevermögen nicht dazu aus, um nach dem Verdunsten der Lösemittel Klebstoffmatrices mit optimaler Wirkstoffbeladung zu ergeben. Selbst in Acrylatklebern, die hauptsächlich aus ethylacetathaltigen Lösemittelgemischen verarbeitet werden, reicht das Lösevermögen nicht aus, um beispielsweise Derivate von Sexualhormonen, wie Ethinylestradiol, Levonorgestrel o.ä., in den Mengen zu lösen, die notwendig wären, einen ausreichenden Konzentrationsgradienten zwischen Transdermalsystem und Haut zu erzeugen.
B) Falls in der Beschichtungslösung ausreichende Wirkstoffkonzentrationen erreicht werden konnten, treten sehr häufig in der Praxis übersättigte und damit metastabile Zustände der eingebetteten Wirkstoffe in der getrockneten Matrix auf. Die Arzneistoffe in solchen metastabilen Matrices tendieren zur Rekristallisation während der Lagerung, wobei die Kristallisation zur Abnahme der Wirkstoffkonzentration und damit zur Abnahme des Konzentrationsgefälles führt. Darüber hinaus können die Klebeeigenschaften der rekristallisierenden Matrices sehr ungünstig, unter Umständen bis zur Unbrauchbarkeit verändert werden. Selbst in Polymeren mit sehr hohem Lösevermögen können z. B. Sexualhormone nicht über 2,5 - 3% stabil gelöst werden. Permeationsexperimente mit stark übersättigten Klebstoffmatrices zeigen, daß die Haut höhere Mengen Hormone permeieren läßt als es stabile gesättigte oder ungesättigte Transdermalsysteme zulassen. Damit ist nicht die Permeationskapazität der Haut der limitierende Schritt, sondern die Wirkstoffbeladung der stabilen Matrices.
   Um nun die Verwendung von - potentiell - schädlichen Absorptionsförderern in Transdermalsystemen zu vermeiden und um eine hohe Fluxrate zu realisieren, die zu zuverlässigen Blutspiegeln führen sollen, besteht ein Bedarf an einer Technologie, mit der Transdermalsysteme mit hoher Wirkstoffbeladung und guter Lagerstabilität herstellbar sind.

Der Einsatz von Wirkstoffeinbettungen in Polymeren zur Modifizierung und Steuerung ihrer Freisetzungseigenschaften ist Stand der Technik. Im Stand der Technik wird die Anwendung von Polymeren zur Stabilisierung von übersättigten Zuständen in TDS beschrieben (J. Pharmacobio-Dyn. 10, 743, 1987). In der DE 4334553 A1 wird mittels eines übersättigten Zustandes eine Erhöhung der Fluxrate erreicht. Diese Übersättigungen in energiereichen Strukturen bergen jedoch bei längerer Lagerung oder längerer Applikationsdauer die Gefahr von Instabilitäten und sind zeitlich begrenzt.

In der EP 0516141 A1 wird eine bioadhäsive pharmazeutische Zubereitung beschrieben, in welcher wirkstofftragende Mikroeinheiten u. a. auch Polymereinbettungen, gemeinsam mit adhäsiven Polymeren und diversen weiteren Hilfsstoffen verpresst und zu Granulen zerkleinert werden. Diese Anwendung enthält zwar den Gedanken, granulenartige Polymereinbettungen unabhängig von der adhesiven Komponente zu verwenden und somit eine größere Flexibilität in der Steuerung der Freisetzungen je nach Anwendung zu erreichen, jedoch ist die hier vorgeschlagene Technologie weder von der Zielrichtung noch von der Verfahrensweise für die Herstellung von stabilen Laminaten für TDS geeignet.

In EP 0481443 A1 werden mikroporöse Partikel bzw. Mikrosphären aus chemisch vernetzten Polymeren als Träger für Wirkstoff und/oder Resorptionsförderer verwendet. Die in der Herstellungstechnologie begründete begrenzte Beladbarkeit der Mikrosphären mit Wirkstoff und der Einsatz von vernetzten Polymeren erzeugen jedoch nur einen begrenzten Diffusionsdruck des Wirkstoffs durch die Haut, so daß auch hier auf die Verwendung von zusätzlichen Resorptionförderem nicht verzichtet werden kann. Diesen Nachteil weisen auch die Systeme auf, die in DE 4405898 A1, EP 0674900 A1 bzw. DE 19701949 A1 beschrieben werden, wo Wirkstoffe gemeinsam mit nichtadhesiven Polymeren verwendet werden, die als Kristallisationsverzögerer dienen bzw. denen nachträglich durch chemische bzw. physikalische Effekte klebende Eigenschaften verliehen werden.

In US-A-5,656,286 wird die Verwendung von löslichem PVC in Mischung mit Polyvinylpyrrolidon zur Verhinderung der Kristallisation von Wirkstoffen beschrieben, ohne jedoch die Wirkstoffabgaberate zu verbessern.

In US-A-5,702,721 wird eine Matrix beschrieben, welche aus einer undurchdringlichen Backing layer, einer Matrix mit aktiver Substanz, die aktiviert werden kann, und einer Schicht, die den Zutritt von Füssigkeit reguliert, besteht. Die Matrix besteht aus einem Material, welches für Wasserdampf permeabel selber aber nicht wasserlöslich ist und keine aktive Substanz enthält. In diese Matrix eingebettet sind "Inseln" von wasserlöslichem oder wasserquellbarem Material, die mit Wirkstoff beladen sind. Die Beladung der unbeladenen Inseln kann durch fest-flüssig Absorption oder über spezielle Trocknungsverfahren vorgenommen werden. Nachteilig ist der komplizierte Aufbau und Herstellungsprozeß dieses mehrschichtigen Systems. Es wird ein besonderer Mechanismus zur Regulierung des Zutrittes der Hautfeuchtigkeit benötigt, da es ansonsten zur Ausfällung des Wirkstoffes bzw. zu ungenügender Wirkstofffreisetzung kommt.

Aufgabe der Erfindung ist es daher, wirkstoffhaltige Laminate für Transdermalsysteme zur Verfügung zu stellen, welche die vorstehend beschriebenen Nachteile des Standes der Technik überwinden, das heißt insbesondere hohe Wirkstoffbeladung und gute Lagerstabilität aufweisen.

Die Aufgabe wird gelöst durch ein Transdermalsystem bestehend aus einem Substrat und einer sexualhormonhaltigen Klebematrix, die Einbettungen des Sexualhormons in einem hydrophilen unvernetzten Polymer in gelöster oder dispergierter Form enthält, wobei die Einbettung eine Sexualhormonkonzentration von 20-90 % aufweist und das Sexualhormon zu mehr als 50 % amorph vorliegt.

Die Herstellung der Wirkstoffeinbettung erfolgt durch an sich bekannte Verfahren, zum Beispiel durch Ausziehen eines Filmes aus Wirkstoff und hydrophilem Polymer und anschließendes Vermahlen oder über Sprühtrocknungs- oder Sprühgranuliertechologie. Erfindungsgemäß ist es bevorzugt, daß der Wirkstoff über Sprühtrocknungs- oder Sprühgranuliertechologie, wie beispielsweise nach dem in der WO 98/26762 beschriebenem Verfahren, im Vergleich zu anderen üblichen Technologien in die polymere Matrix mit hoher Beladung und in thermodynamisch hochaktiver Form als feste Lösung eingebettet und mit oder ohne Träger als mikrofeines Pulver hergestellt wird.

Es ist erfindungsgemäß bevorzugt, daß der Wirkstoff in der Einbettung zu mehr als 50 Gew.-% in amorpher Form vorliegt. Besonders bevorzugt ist hierbei, daß der Wirkstoff in der Einbettung zu mehr als 95 Gew.-% in amorpher Form vorliegt. Die Messung und Quantifizierung der Kristallinität des Wirkstoffes erfolgt in an sich bekannter Weise durch Röntgenpulverdiffraktometrie(XRPD) über Vergleichsmessung mit der entsprechenden, kristallinen Modifikation des Wirkstoffes.

Erfindungsgemäß bevorzugt ist ferner, daß die Konzentration des Wirkstoffes in der Einbettung 20 bis 90 Gew.-%, besonders bevorzugt 20 bis 40 Gew.-% beträgt.

Das erfindungsgemäße wirkstoffhaltige Laminat für Transdermalsysteme zeichnet sich ferner dadurch aus, daß die Wirkstoffeinbettung in gelöster Form in eine Klebematrixlösung eingebracht ist. Erfindungsgemäß ist es aber auch, daß die Wirkstoffeinbettung als feste Partikel in der Klebematrix feindispers und homogen eingebracht ist.

Die in dem erfindungsgemäßen Laminat enthaltenen Wirkstoffe sind Sexualhormone, wie Estrogene, beispielsweise Estradiol, Estradiolester, wie Estradiolvalerat, Estradiol-3-benzoat, Estradiol-17-valerat, -cypionate, -undecylate und -enanthate, Estradiol-, Ethinylestradioland Estron-3-sulfamates, beispielsweise Estron-N,N-dimethylsulfamat, Estron-N,N-diethylsulfamat, Ethinylestradiol-3-N,N-dimethylsulfamat, Ethinylestradiol-3-N,N-diethylsulfamat, Ethinylestradiol-3-N,N-tetramethylensulfamat, Estronsulfamat, Estradiol-3-sulfamat, Estradiol-3-N,N-dimethylsulfamat, Estradiol-3-N,N-diethylsulfamat, Ethinylestradiol-3-sulfamat (DE 44 29 398 A1 and DE 44 29 397 A1), Gestagene, wie Dienogest, Desogestrel und Drospirenon, Antigestagene, die beispielsweise in DE 43 32 284, DE 43 32 283 und 198 09 845.6 beschrieben sind, wie beispielsweise [4-[17β-Methoxy-17α-(methoxymethyl)-3-oxoestra-4,9-dien-11β-yl]benzaldehyd-(1E)-oxim], [4-[17β-Hydroxy-17α-(methoxymethyl)-3-oxoestra-4,9-dien-11β-yl]benzaldehyd-(1E)-oxim], [4-[17β-Methoxy-17a-(methoxymethyl)-3-oxoestra-4,9-dien-11β-yl]benzaldehyd-(1E)-[O-(ethoxy)carbonyl]oxim], [4-[17β-Methoxy-17α-(methoxymethyl)-3-oxoestra-4,9-dien-11β-yl]benzaldehyd-(1E)-(O-acetyl)oxim], [4-[17β-Methoxy-17α-(methoxymethyl)-3-oxoestra-4,9-den-11β-yl]benzaldehyd-(1E)-[O-(ethylamino)carbonyl]oxim], [4-[17β-Methoxy-17α-(methoxymethyl)-3-oxoestra-4,9-dien-11β-yl]benzaldehyd-(1E)-[O-(ethylthio)carbonyl]oxim und Androgene, wie Testosteron, Testosteronundecanoat und Dehydroepiandrosteron (DHEA).

Erfindungsgemäß wird das hydrophile Polymer aus der Gruppe der unvernetzten hydrophilen Polymere ausgewählt, wobei das Polymer zur Einbettung insbesondere aus der Gruppe der Polyvinylpyrrolidone, Methylcellulosen, Ethylcellulosen, Hydroxypropylcellulosen oder deren Mischungen ausgewählt wird.
Erfindungsgemäß bevorzugt ist auch, daß die Klebematrix aus der Gruppe der Polyisobutylene, Ethylen-Vinyl-Copolymere (poly-EVA), Polystyrol-Butadien-Blockcopolymere oder deren Mischungen oder aus der Gruppe der druckempfindlichen Haftkleber auf Acrylat- oder Siliconbasis ausgewählt ist.
Überraschenderweise wurde gefunden, daß die Verwendung von hochkonzentrierten Wirkstoffeinbettungen in nichtadhäsive Polymere, wie Polyvinylpyrrolidon, Ethylcellulose, Hydroxypropylcellulose o. ä., die oben beschriebenen Probleme des Standes der Technik gleichzeitig lösen kann.
In der erfindungsgemäßen Technologie werden speziell konditionierte hochkonzentrierte Wirkstoffeinbettungen in Polymeren derart verwendet, daß hohe Konzentrationen vor allem bei sich in der haftklebenden Patchmatrix schlecht lösenden Wirkstoffen erreicht werden können, ohne daß unerwünschte Rekristallisationen wie im Falle übersättigter Lösungen eintreten. Die Verwendung von derartigen hochbeladenen, thermodynamisch hochaktiven Wirkstoff-Polymerkombinationen in einem einfach herzustellenden einschichtigen und ohne Resorptionsförderer auskommenden TDS mit dem Ziel der maximalen Erhöhung des Konzentrationsgradienden zwischen TDS und Haut ist in der Literatur bisher nicht beschrieben.

Während in der EP 0516141 A1 eine feste, bioadhäsive Mischung mit einer entsprechend der jeweiligen Applikation retardierten Freisetzung beschrieben wird, zielt die erfindungsgemäße Technologie auf höchstmögliche und stabile Wirkstoffkonzentrationen in der adhäsiven Laminat-Matrix vor und während der Applikation.

Diese kann erfindungsgemäß auf zwei Wegen erreicht werden.
A) Während das gestagene Hormon Gestoden sich nur zu 3 % in Ethylacetat löst, kann beispielsweise unter Verwendung einer 20 %-igen festen Einbettung von Gestoden in Polyvinylpyrrolidon die Löslichkeit auf 10-15% in Ethylacetat/Polymer-Mischungen ohne Rekristallisierung gesteigert werden. Dementsprechend können unter Verwendung der festen Polymereinbettungen die jeweils optimalen Wirkstoffbeladungen diverser Polymere erzielt werden. Die Wirkstoffkonzentrationen in den Polymereinbettungen können auf sehr hohem Niveau stabilisiert werden, so daß nur minimale Mengen zusätzlicher Lösemittel benötigt werden. In den Fällen, in denen die Löslichkeit der Arzneistoffe in den Klebstoffen der die Hautpermeation limitierende Faktor ist, muß die Herstellungsmethode, wie unter B) beschrieben, gewählt werden.
B) Die Wirkstoffpermeation kann weiter gesteigert werden, wenn die hochkonzentrierten Wirkstoff-Polymereinbettungen ihrerseits in Klebstoffe eingebettet werden, in denen sie sich nicht lösen. Solche Matrices werden z. B. durch Mischen von mikronisierten Wirkstoff-Polymereinbettungen in Polyvinylpyrrolidon mit einer Polyisobutylen/Harzmischung in n-Heptan hergestellt. Hierin wirkt der Klebstoff nur als Fixierhilfsmittel für die Polymereinbettung auf der Haut. Die Wirkstoffkonzentrationen in den Polymereinbettungen können 50 Gew.-% deutlich überschreiten.
Die Verwendung von festen Wirkstoffeinbettungen in geeigneten nicht-adhäsiven Polymeren ist Voraussetzung für die Erzielung hoher Wirkstoffkonzentrationen in der adhäsiven Klebematrix unter Vermeidung von Rekristallisationserscheinungen. Als nicht-adhäsive Polymere kommen Polyvinylpyrrolidone, Methylcellulose, Ethylcellulose, Hydroxypropylcellulose und andere in Frage. Diese Aufzählung soll keine Einschränkung darstellen. Diese festen Wirkstoffeinbettungen können in der adhesiven Matrix in gelöster oder in dispergierter, fester Form vorteilhaft zum Einsatz gebracht werden. Die Beladung der festen Polymereinbettungen mit Wirkstoff wird bestimmt durch die oben genannten Einsatzfälle und die Permeationsanforderungen. Sie kann je nach Art der verwendeten Wirkstoff-Polymerkombination größer als 50% sein und ist vollständig oder überwiegend amorph.

Werden solche festen Wirkstoffeinbettungen in gelöster Form in adhäsiven Matrixlösungen zum Einsatz gebracht, ist die gute Löslichkeit des Einbettungspolymers im verwendeten Matrix-Lösungsmittel, wie Ethylacetat, Ethanol u. a. von Bedeutung. Es wird eine schnelle Auflösung des amorphen Wirkstoffes weit oberhalb seiner Sättigungslöslichkeit im reinen Lösungsmittel erreicht. So löst beispielsweise Ethylacetat bei Raumtemperatur nur etwa 3 Gew.-% eines gestagenen Steroids. Bei Verwendung einer entsprechenden festen Wirkstofflösung konnte die Löslichkeit des Steroids in der Lösung von Ethylacetat und nichtadhäsiven Polymer auf nahezu 15 Gew.-% gesteigert und stabil gehalten werden. Dabei ist zu beachten, daß bezogen auf das flüchtige, aus der adhäsiven Matrix abzutrocknende Lösungsmittel Ethylacetat, die Löslichkeit auf über 30 Gew.-% gesteigert wurde. Das Polymer inhibiert dabei die Kristallisation in der stark übersättigten adhäsiven Lösung und anschließend nach der Trocknung in der adhäsiven Wirkstoffmatrix.

Die Höhe der zulässigen Wirkstoffbeladung ist abhängig von dieser Inhibitorwirkung, die wiederum beeinflußt wird von der Wahl des Einbettungs-Polymers, dem Wirkstoff selbst und dem verwendeten polaren Lösungsmittel. Der Inhibitionseffekt sinkt mit wachsendem Wirkstoffanteil. Beispielsweise führten im Falle eines gestagenen Steroids Beladungen von größer als 20% in einem Polyvinylpyrrolidon zu Rekristallisationserscheinungen in der Ethylacetatlösung. Bei Verwendung eines estrogenen Steroids waren im gleichen System wesentlich höhere Beladungen möglich.
Ein weiterer wichtiger Punkt ist der Grad der Amorphizität des Wirkstoffes in der festen Polymereinbettung. Besonders bevorzugt beträgt er nahezu 100% oder 100%, da ein Wirkstoff mit Restkristallinität sich in der übersättigten Lösung nur unvollständig lösen und die Rekristallisation fördern kann. Die Korngrößenverteilung ist für diese Anwendung ohne Relevanz.

Anders verhält es sich bei der Verwendung von festen Wirkstoffeinbettungen, die als feste Partikel im druckempfindlichen adhäsiven Polymer feindispers und homogen verteilt werden. Diese festen Wirkstoffeinbettungen sind in der adhäsiven Matrixlösung nahezu unlöslich. Ihre Beladung mit Wirkstoff wird je nach Permeationsanforderungen eingestellt und ist lediglich durch die Forderung begrenzt, daß der Wirkstoff möglichst vollständig, zumindest aber überwiegend, amorphisiert vorliegen sollte und daß dieser amorphe Anteil während der Lagerung, Verarbeitung und als Dispersion im adhäsiven Polymer stabil bleibt, d. h. nicht rekristallisiert. Je nach Art der verwendeten Kombination Wirkstoff/Polymer ist ab einer bestimmten Beladung die Kapazität des Polymers für einen stabilen amorphen Status des Wirkstoffes erschöpft. Beispielsweise konnten mit einer Hydroxypropylcellulose und Kollidon stabile, überwiegend amorphe Einbettungen von estrogenen und gestagenen Steroiden mit bis zu 80% Wirkstoff erzeugt werden. Da die Inhibitorfunktion bezüglich der Rekristallisation des im adhäsiven Polymer gelösten Wirkstoffes für diese Anwendung entfällt, kann die Amorphisierungskapazität des Polymers voll ausgeschöpft werden. Damit wird, wenn erforderlich, eine sehr hohe Konzentration des amorphisierten und feindispers verteilten Wirkstoffes in der adhäsiven Patchmatrix möglich, ohne daß die Gefahr der Rekristallisation besteht.
Weiterhin bietet sich mit diesem Verfahren die Möglichkeit, chemisch empfindliche, instabile Wirkstoffe bis zum Zeitpunkt ihrer Permeation schützend einzubetten oder aber mehrere Wirkstoffe mit unterschiedlichen Permeationsanforderungen isoliert voneinander, sozusagen "tailormade", einzubetten und in einem Patch zur Wirkung zu bringen.

Die durchschnittliche Partikelgröße der festen Polymereinbettungen ist kleiner als 10 µm, vorzugsweise kleiner als 5 µm, bewegt sich also im Bereich von mikronisierten Wirkstoffen. Die Herstellung dieser festen Polymereinbettungen erfolgt mit den üblichen Verfahren der pharmazeutischen Technologie.

Erfindungsgemäß werden die festen Wirkstoff-Polymer-Einbettungen zur Erhöhung der Wirkstoffkonzentration in Beschichtungslösungen verwendet.
Lösungen von druckempfindlichen Haftklebern auf Acrylatbasis, die von verschiedenen Herstellern erhalten werden können (beispielhaft seien die Duro-Tak-Kleber von National-Starch & Chemical Corporation oder verschiedene Gelva-Typen von Monsanto oder Silikon-Kleber wie Bio-PSA von Dow Corning o. ä. genannt), werden homogen mit hochkonzentrierten Wirkstofflösungen in mittelpolaren Lösemitteln, wie Ethylacetat oder Alkoholen, gemischt.
Diese hochkonzentrierten Lösungen erhält man durch Auflösen von festen Polymer-Wirkstoffeinbettungen in nichtadhäsiven Polymeren, deren Herstellung beschrieben wurde.
Die erhaltenen Lösungen werden eventuell durch weitere Zusätze auf die erforderliche Viskosität und/oder Feststoffgehalt eingestellt. Die Lösungen können mit herkömmlichen, jedem Fachmann bekannten Beschichtungsverfahren, z. B. Rakelauftrag, Umkehrwalzenauftrag, Düsenauftrag o. ä., auf silikonisierte Folien, Papiere oder dergl. aufgetragen werden. Das beschichtete Substrat wird in Trocknungseinrichtungen, wie Trocknungstunneln oder Trocknungsöfen von den flüchtigen Lösemitteln befreit, und die entstehende selbstklebende Matrix wird mit einer Abdeckfolie, -gewebe, -vlies oder dergleichen kaschiert. Das Laminat wird aufgerollt und entweder in schmalere Rollen geschnitten oder direkt in üblichen Stanzen oder Schneidanlagen zu definierten Einzelstücken, den Transdermalsystemen, gestanzt oder geschnitten. Erfindungsgemäß ist keine Schicht erforderlich, die den Eintritt der kutanen Flüssigkeit steuert und der Haut gegenüberliegt.
Zur geschützten Lagerung können die Transdermalsysteme in Beutel gepackt werden. Jedes der oben angegebenen Polymere (seien es Acrylate, Silikone, o. ä.) weist unterschiedliche Lösungskapazitäten für Wirkstoffe entsprechend ihrer Zusammensetzung auf. Durch Anwendung der erfindungsgemäßen Herstellungstechnologie können die Sättigungskonzentrationen der betreffenden Wirkstoffe in unterschiedlichen druckempfindlichen Haftklebern erzielt werden.
Damit können, in Bezug auf die jeweiligen Haftkleber, optimierte Wirkstofffluxe erreicht werden.

Erfindungsgemäß werden die festen Wirkstoff-Polymer-Einbettungen auch in Kombination mit inerten druckempfindlichen Haftklebern verwendet.
Die wirkstoffhaltigen Polymereinbettungen sind vorstehend beschrieben. Die festen Wirkstoffeinbettungen werden pulverförmig in unterschiedlicher Korngröße, bis zu mikronisierten Pulvem, verwendet. Die pulverförmigen Einbettungen werden in apolaren Lösemitteln, wie Hexan, Heptan oder dergl. suspendiert und mit apolaren Lösungen von druckempfindlichen Haftklebern, wie Polyisobutylen, Poly-EVA, Polystyrol-Butadien-Blockcopolymeren oder dergl. homogen gemischt. Die Lösungen können weitere übliche Zusätze wie Antioxidantien, Klebeharze, Weichmacher, Lösungsvermittler o.ä. enthalten, die zur Herstellung stabiler, gut klebender und hautverträglicher Transdermalsysteme nötig sind. Die festen Wirkstoff-Polymereinbettungen sollen sich nicht wesentlich in diesen Lösemitteln auflösen. Die Pulver werden bis zur Homogenität mit den Kleberzubereitungen gemischt. Beschichtung, Trocknung und Laminieren erfolgt wie oben beschrieben.
Die erhaltene Matrix besteht aus dem druckempfindlichen haftklebenden Polymer, das als Fixierelement des Transdermalsystems auf der Haut wirkt, dem Wirkstoffreservoir, als gleichmäßig verteilte Wirkstoff-Polymereinbettungspartikel. Das klebende Polymer sollte idealerweise nicht mit der Polymereinbettung wechselwirken.
Wenn diese Voraussetzungen gegeben sind, wird die Wirkstoffpermeation durch Membranen aus den erfindungsgemäßen Transdermalsystemen im wesentlichen durch die Art des Polymers der Wirkstoffeinbettung und durch die Wirkstoffkonzentration in der Einbettung bestimmt. Hierdurch körnen Wirkstoffkonzentrationen in Transdermalsystemen (TDS) erreicht werden, die solche, die in den Klebstoffen direkt erzielbar sind, bei weitem übersteigen. Da die Wirkstoffe nicht in den Klebstoffen gelöst sind, bei der Lagerung die Klebstoffe aber nur Wirkstoff entsprechend ihrer maximalen Löslichkeit aufnehmen können, besteht nicht die Gefahr der Rekristallisation der herkömmlichen Systeme.

Die Wirkstoffpermeation kann durch unterschiedliche Maßnahmen kontrolliert werden:
- Partikelgröße der Wirkstoff-Polymereinbettung
- Wirkstoffkonzentration in der Wirkstoff-Polymereinbettung
- Partikelanzahl der Wirkstoff-Polymereinbettung pro Volumenelement des Klebers
- Freisetzungsfläche des Transdermalsystemes
- Löslichkeit des Wirkstoffes im Einbettungspolymer
- Interaktion der Wirkstoff-Polymereinbettung mit Adsorbatwasser aus der Haut
- Kristallinität des Wirkstoffes in der Wirkstoff-Polymereinbettung
Erfindungsgemäße Transdermalsysteme zeichnen sich bei einem hohen Wirkstoffflux ohne Absorptionsbeschleuniger dadurch aus, daß in den Partikeln der Wirkstoff-Polymer-Einbettung eine hohe Wirkstoffkonzentration erhalten werden kann, die zum Teil über 80 % betragen kann. Die Polymere, die für die Einbettung verwendet werden, können entsprechend ihren besten Stabilisierungen hoher Wirkstoffmengen unabhängig von ihrer Klebkraft, kaltem Fluß o.ä., ausgewählt werden. Demzufolge können unerwünschte Rekristallisierungen, selbst bei Konzentrationen über 50 Gew.-% vermieden werden.
Die Wirkstoffpolymereinbettung wird in der polymeren Klebematrix dispergiert, ohne sich darin aufzulösen. Das heißt, in fertigen Matrixsystemen sind die Eigenschaften der hochkonzentrierten Polymereinbettung im wesentlichen erhalten, wodurch ein hohes Wirkstoffgefälle zwischen der Polymereinbettung und der Haut erzeugt wird, das viel größer ist, als wenn der Wirkstoff gleichmäßig in einer Mischung aus Einbettungspolymer und Klebematrix dispergiert ist. Wenn beispielsweise 10 mg einer 80 %-igen Wirkstoffeinbettung in 90 mg Klebematrix dispergiert werden, beträgt die Wirkstoffkonzentration nach wie vor 80 % innerhalb der Wirkstoffeinbettung, wohingegen bei einer vollständigen Lösung des Wirkstoffes in der Gesamtmatrix die Konzentration nur 8 % beträgt.
Die folgenden Beispiele erläutern die Erfindung.

### Beispiel 1

Feste Wirkstoff-Polymereinbettung zur Erhöhung der Konzentration des in der adhäsiven Matrix gelösten Wirkstoffes:
20 % Gestoden
80 % Kollidon
Korngrößenverteilung:
   100% < 103 µm
   50% < 24 µm
   10% < 4,3 µm
Amorphizität des Wirkstoffes: 100% (XRPD)

### Beispiel 2

Feste Wirkstoff-Polymereinbettung zur Herstellung einer homogenen feindispersen Suspension in der adhäsiven Matrix:
30% Gestoden
70% Kollidon
Korngrößenverteilung:
   100% < 21 µm
   50% < 3,7 µm
   10% < 1,1 µm
Amorphizität des Wirkstoffes 100% (XRPD)

### Beispiel 3

Transdermalsystem mit einer homogenen, feindispersen Suspension in der adhäsiven Matrix
10 g der unter Beispiel 2 beschriebenen Polymereinbettung werden in 169,7 g eines handelsüblichen Polyisobutylenklebers homogen dispergiert. Die Dispersion wird auf eine silikonisierte Polyesterfolie einer Stärke von 80 µm mittels üblicher Beschichtungsverfahren, wie Rakelauftrag, aufgebracht und getrocknet. Nach dem Trocknen resultiert eine selbstklebende Matrix mit einem Flächengewicht von 100 g/m², bei einem Gehalt von 5,1 % Gestoden in der Matrix. Die selbstklebende Matrix wird mit einer Abdeckfolie (backing foil) von z. B. 19 µm Stärke abgedeckt und das Laminat zu Transdermalsystemen mit z. B. 10 cm² Fläche verarbeitet. Ein derartig gefertigtes Transdermalsystem weist eine in vitro Hautpermeation von 40,3 µg Gestoden/cm²/24 h auf, gemessen in Diffusionszellen an intakter Haut von Nacktmäusen.

### Beispiel 4

Feste Wirkstoff-Polymereinbettung hergestellt nach folgendem Verfahren:

100 g Gestoden und 150g Kollidon (VA64) werden in 2360 g Ethanol gelöst und die Lösung bei 64 bis 65°C bei 32 ml Lösung je m³ Trocknungsgas sprühgetrocknet. Es wird ein mikrofeines Pulver mit folgenden Parametern erhalten:
Partikelgrößenverteilung:
   100% < 20 µm
   90% < 6.4 µm
   50% < 2.8 µm
   10% < 1.0 µm

- XRPD:: 100% amorph
- Ethanol:: 0.84%

## Patentansprüche

1. Transdermalsystem bestehend aus einem Substrat und einer sexualhormonhaltigen Klebematrix, die Einbettungen des Sexualhormons in einem hydrophilen unvernetzten Polymer in gelöster oder dispergierter Form enthält, wobei die Einbettung eine Sexualhormonkonzentration von 20-90 % aufweist und das Sexualhormon zu mehr als 50 % amorph vorliegt.

2. Transdermalsystem nach Anspruch 1, **dadurch gekennzeichnet, dass** das Sexualhormon in der Einbettung zu mehr als 95 % Gew.% in amorpher Form vorliegt.

3. Transdermalsystem gemäß Anspruch 1 oder 2, **dadurch gekennzeichnet, dass** die Sexualhormoneinbettung in gelöster Form in der Klebematrix vorliegt.

4. Transdermalsystem gemäß Anspruch 1 oder 2, **dadurch gekennzeichnet, dass** die Sexualhormoneinbettung in dispergierter Form in der Klebematrix vorliegt.

5. Transdermalsystem gemäß einem der Ansprüche 1 bis 4, **dadurch gekennzeichnet, dass** als unvernetztes hydrophiles Polymer Polyvinylpyrrolidon, Methylcellulose, Ethylcellulose, Hydroxyethylcellulose oder deren Mischungen verwendet wird.

6. Transdermalsystem nach einem der Ansprüche 1 bis 5, **dadurch gekennzeichnet, dass** als Klebematrix Polyisobutylen, Ethylen-Vinyl-Copolymere (poly-EVA), Polystyrol-Butadien-Blockcopolymere oder deren Mischungen verwendet wird.

## Claims

1. Transdermal system consisting of a substrate and of a sex hormone-containing adhesive matrix which comprises embeddings of the sex hormone in a hydrophilic uncrosslinked polymer in dissolved or dispersed form, where the embedding has a sex hormone concentration of 20-90%, and the sex hormone is more than 50% in amorphous form.

2. Transdermal system according to Claim 1, **characterized in that** the sex hormone present in the embedding is more than 95% in amorphous form.

3. Transdermal system according to Claim 1 or 2, **characterized in that** the sex hormone embedding is present in dissolved form in the adhesive matrix.

4. Transdermal system according to Claim 1 or 2, **characterized in that** the sex hormone embedding is present in dispersed form in the adhesive matrix.

5. Transdermal system according to any one of Claims 1 to 4, **characterized in that** polyvinylpyrrolidone, methylcellulose, ethylcellulose, hydroxyethylcellulose or mixtures thereof is used as uncrosslinked hydrophilic polymer.

6. Transdermal system according to any one of Claims 1 to 5, **characterized in that** polyisobutylene, ethylene-vinyl copolymers (poly-EVA), polystyrene-butadiene block copolymers or mixtures thereof is used as adhesive matrix.

## Revendications

1. Système transdermique se composant d'un substrat et d'une matrice adhésive contenant une hormone sexuelle, qui contient des inclusions de l'hormone sexuelle dans un polymère hydrophile non réticulé sous une forme dissoute ou dispersée, l'inclusion présentant une concentration de l'hormone sexuelle de 20-90% et l'hormone sexuelle existant à raison de plus de 50% sous une forme amorphe.

2. Système transdermique selon la revendication 1, **caractérisé en ce que** l'hormone sexuelle se présente dans l'inclusion jusqu'à plus de 95% en poids sous une forme amorphe.

3. Système transdermique selon la revendication 1 ou 2, **caractérisé en ce que** l'inclusion de l'hormone sexuelle existe dans la matrice adhésive sous forme dissoute.

4. Système transdermique selon la revendication 1 ou 2, **caractérisé en ce que** l'inclusion de l'hormone sexuelle existe dans la matrice adhésive sous forme dispersée.

5. Système transdermique selon l'une quelconque des revendications 1 à 4, **caractérisé en ce que** l'on utilise, en tant que polymère hydrophile non réticulé, la polyvinylpyrrolidone, la méthylcellulose, l'éthylcellulose, l'hydroxyéthylcellulose ou leurs mélanges.

6. Système transdermique selon l'une quelconque des revendications 1 à 5, **caractérisé en ce que** l'on utilise, en tant que matrice adhésive, le polyisobutylène, des copolymères éthylène-vinyle (poly-EVA), des copolymères séquencés polystyrène-butadiène ou leurs mélanges.
